# EUROPEAN PATENT SPECIFICATION

(11) **EP 0 880 956 B1**
(45) Date of publication and mention of the grant of the patent: **08.10.2003**
(21) Application number: 98304253.2
(22) Date of filing: 29.05.1998
(51) Int. Cl.: A61F 13/15

(54) **Disposable absorbent garment with extendible fastening means**
Absorbierender Wegwerfartikel mit dehnbaren Befestigungsmitteln
Vêtement à jeter avec moyens de fermeture extensible

(30) Priority: 30.05.1997 JP 14263097
(43) Date of publication of application: 02.12.1998
(73) Proprietor: UNI-CHARM CORPORATION, Kawanoe-shi Ehime-ken (JP)
(72) Inventor: Onishi, Kazuaki, Mitoyo-gun, Kagawa-ken 769-1602 (JP)
(74) Representative: Parry, Christopher Stephen

(56) References cited:
- WO-A-95/13775
- WO-A-96/21412
- US-A- 4 701 174

## Description

This invention relates generally to disposable garments such as disposable diapers or training pants including a pair of wings provided with fastener means for separably coupling front and rear waist regions of the garment.

US-A-4,701,174 discloses a disposable garment according to the preamble of Claim 1. comprising a main body assembled from a liquid-impermeable and resiliently stretchable outer cover and a liquid-absorbent structure attached to the outer cover, said stretchable outer cover having front and rear waist regions with respect to said longitudinal direction and a crotch region having curved outer edges extending between the outer edges of the waist regions; and fastener means attached to respective outer edges of the front and rear waist regions for separably coupling said front and rear waist regions to each other.

Japanese Laid-Open Utility Model Application No. Hei5-65321 also discloses a disposable diaper. Each of the wings is provided with elastic members bonded thereto under appropriate tension. With this diaper, undesirable gathers are formed in the wings as the elastic members contract. Such gathers inevitably give a wearer uncomfortably rough touch when these gathers come in contact with the wearer's skin. In addition, the upper and lower edges of the wings extending along the elastic members are liable to be curved outwardly of the wearer's body. These edges thus curved outward readily come in contact with the wearer's arms, not only obstructing free movement of the arms but also deteriorate the appearance of the diaper.

In view of the problems as has been described above, it is a principle object of the invention to provide a disposable diaper allowing both the gathers and the outward curving which have conventionally appeared in the wings to be eliminated and thereby allowing a feeling to wear the diaper as well as the appearance to be improved.

The object set forth above is achieved, according to the invention, by a disposable garment comprising a main body having a transverse direction defining the wearer's waist regions and a longitudinal direction orthogonal to said transverse direction, said main body being assembled from a liquid-permeable topsheet, a liquid-impermeable backsheet and a liquid-absorbent core disposed between these two sheets, said main body having front and rear waist regions with respect to said longitudinal direction and a crotch region extending between these waist regions; said main body having side edges opposed to each other in the transverse direction and a pair of wings extending outward of said main body in said transverse direction from the side edges of the main body in said rear waist region; and fastener means attached to respective distal ends of said wings for separably coupling said front and rear waist regions to each other; characterised in that said wings are formed separately from the main body, and fixed thereto by bonding means and respectively comprise layer members being extendible in said transverse direction and elastically stretchable strips placed along upper and lower edges of said layer members extending in said transverse direction without tension and having a stretch stress higher than that of said layer members; and said fastener means are non-stretchable and more rigid than said wings wherein said fastener means are attached to said distal ends of said wings so as to extend in said longitudinal direction and to overlap said elastically stretchable strips on both the upper and lower edges of said layer members.

Preferably, said fastener means extend in said longitudinal direction across entire widths of said wings and said elastically stretchable strips comprise hot metal adhesive.

Embodiments of the invention are described below.
Fig. 1 is a plan view showing a disposable garment according to the invention implemented in the form of a disposable diaper as partially broken away;
Fig. 2 is a perspective view showing the diaper as in a configuration when it is put on the wearer's body; and
Fig. 3 is a fragmentary scale-enlarged view showing an important part of Fig. 1 as partially broken away.

Details of a disposable garment will be more fully understood from the description of a disposable diaper as a specific embodiment of the invention given hereunder with reference to the accompanying drawings.

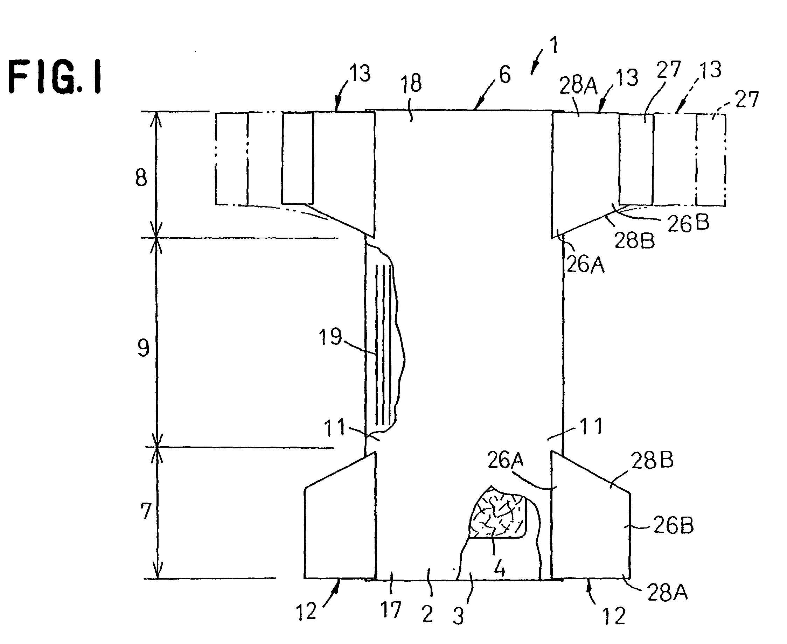

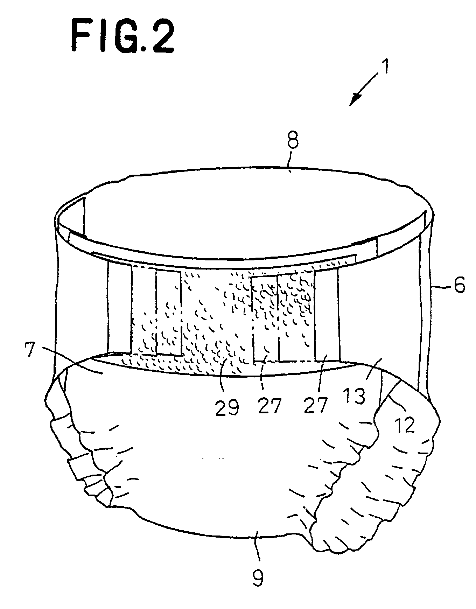

Fig. 1 is a plan view showing an inner side of a disposable diaper as partially broken away and Fig. 2 is a perspective view showing the diaper 1 as assembled for actual use.

The diaper 1 generally comprises a main body 6, a pair of front wings 12 and a pair of rear wings 13 extending laterally from said main body 6. The main body 6 has a transverse direction, i.e., a circumferential direction with respect to the wearer, a longitudinal direction which is orthogonal to the transverse direction and a thickness direction which is perpendicular to the plane defined by Fig. 1. In the thickness direction, the main body 6 comprises a laminate consisting of a liquid-permeable topsheet 2, a liquid-impermeable backsheet 3 and a liquid-absorbent core 4 disposed between these two sheets 2, 3. In its configuration, the main body 6 has a front waist region 7, a rear waist region 8 and a crotch region 9 extending between these waist regions 7, 8. Both the front and rear waist regions 7, 8 are provided with the pair of front wings 12 and the pair of rear wings 13 extending laterally from transversely opposite side edges of these waist regions 7, 8. Each of these front and rear wings 12, 13 is defined by a proximal end 26A, a distal end 26B, an upper edge 28A and a lower edge 28B. The lower edge 28B obliquely extends so that the wing may be tapered from the proximal end 26A toward the distal end 26B. The respective wings 12, 13 have their proximal ends 26A fixed to the main body 6 by well known bonding means such as an adhesive agent or heal-sealing technology (not shown) so that these wings 12, 13 may form transversely opposite side portions of the waist regions as the diaper 1 is put on the wearer's body. The pair of rear wings 13 are adapted to be extendible transversely of the main body 6, as indicated by imaginary lines in Figs. 1 and 2. The pair of front wings 12 may be selectively arranged to be extendible or not, transversely of the main body 6.

Regarding the main body 6, the topsheet 2 and the backsheet 3 extend outward beyond a peripheral edge of the absorbent core 4 and are bonded together along these extensions by means of a hot melt adhesive agent (not shown) so as to form transversely opposite side flaps 11 and longitudinally opposite ends 17, 18. Each of the side flaps 11 at least in the crotch region 9 includes a plurality of elastic members 19 longitudinally extending between the topsheet 2 and the backsheet 3 and bonded under their longitudinally stretched conditions to an inner surface of at least one of the topsheet 2 and the backsheet 3.

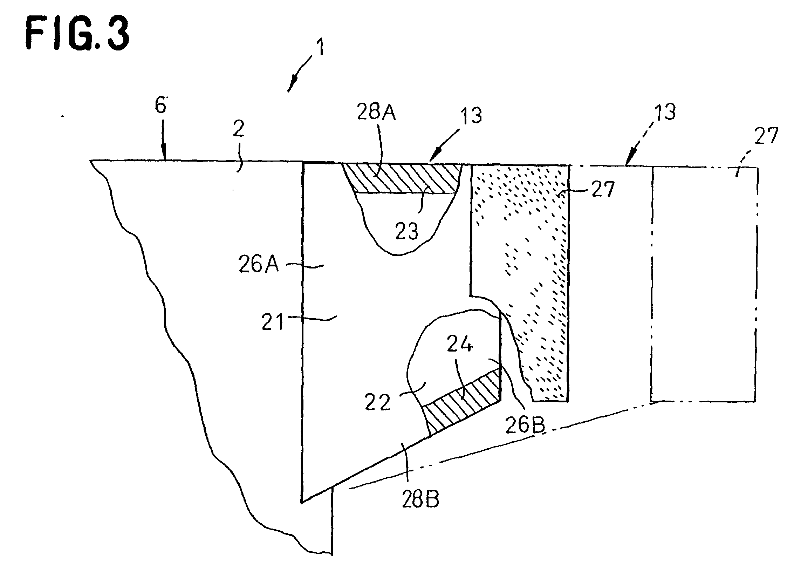

Fig. 3 is a fragmentary plan view showing, in an enlarged scale, an important part of Fig. 1. As shown, the rear wing 13 is stretchable at least transversely of the diaper 1. More specifically, the rear wing 13 comprises an inner layer member 21 intended to come in contact with the wearer's skin, an outer layer member 22 opposed to the inner layer member 21, first and second elastically stretchable strips 23, 24 disposed between these the inner and outer layer members 21, 22 so that the first and second elastically stretchable strips 23, 24 function also as adhesive means by which the inner and outer layer members 21, 22 are bonded together, and a hook fastener 27 attached to the distal end 26B of the wing 13. The hook fastener 27 makes a part of so-called mechanical fastener means consisting of hook and loop fasteners commonly known under the trademark "VELCRO". The loop fastener 29 cooperating with the hook fastener 27 is attached to an outer surface of the front waist region 7 in a landing zone of the hook fastener 27 (see Fig. 2).

The inner and outer layer members 21, 22 may be of a sheet material which is extendible transversely of the diaper 1 by 30 % or more, preferably 50 % or more and more preferably 100 % or more. Such sheet material may be replaced by a sheet material which is elastically stretchable by 30 % or more, preferably 50 % or more and more preferably 100 % or more. Such sheet material may be extendible or elastically stretchable a nonwoven fabric, an elastomer film, a rubber film or the like. The inner and outer layer members 21, 22 are bonded together not only by means of the first and second elastically stretchable strips 23, 24 but also by means of an adhesive agent intermittently applied on the inner and outer layer members 21, 22 or intermittently formed heat-sealing spots on these members 21, 22 so that the extendibility or elastic stretchability of these members 21, 22 is not affected by bonding of these members 21, 22. The inner and outer layer members 21, 22 are preferably of breathable nature.

The first and second elastically stretchable strips 23, 24 are provided along upper and lower edges 28A, 28B, respectively, of each rear wing 13, both extending transversely of this wing 13, to make these edges elastically stretchable. The first and second elastically stretchable strips 23, 24 are made of material selected so that, when a sample strip of this material is stretched by 50 % transversely of the diaper 1, it generates a stress higher than a stress generated by the inner and outer layer members 21, 22 under the same condition. At the same time, this material should have an elastically stretchability of 50 % or higher and more preferably 100 % or higher, transversely of the diaper 1. An example of such material is a hot melt adhesive agent made of ethylene elastomer- or urethane-based polymer, which may be applied to an inner surface of any one of the inner and outer layer members 21, 22. This hot melt adhesive agent is disposed between the inner and outer layer members 21, 22 under no tension and thus the inner and outer layer members 21, 22 are bonded together with no tension. Alternatively, a strip of elastically stretchable film such as an elastomer film may be used as said material of the first and second elastically stretchable strips 23, 24. In this case also, the strip of film and the inner and outer layer members 21, 22 are bonded together with no tension so that they may be free to be elastically stretched. Such unique manner in which the inner layer member 21, the outer layer member 22, the first elastically stretchable strip 23 and second elastically stretchable strip 24 are bonded together is effective to avoid the formation of gathers in the rear wing 13 no matter what material is employed for the first and second elastically stretchable strips 23, 24.

The fastener 27 is preferably attached to the distal end 26B of the rear wing 13 so as to extend vertically of the rear wing 13 and to overlap both the first elastically stretchable strip 23 and the second elastically stretchable strip 24. While the fastener 27 is shown to be attached to the inner layer member 21 defining an inner side of the diaper 1, the fastener 27 may be attached any one of the inner and outer layer members 21, 22. The fastener 27 should have a rigidity higher than that of the rear wing 13 and should be non-stretchable. When the rear wings 13 are pulled toward the front waist region 7 with such fasteners 27 held by a wearer or a helper, the resultant tension uniformly propagates over an entire width of each rear wing 13 without any local concentration of said tension. Accordingly, the rear wings 13 and the end flap 18 of the diaper 1 connected to the wings 13 are not curved outwardly of the wearer's body even after the fasteners 27 have been anchored on the respective loop fasteners 29 provided on the front waist region 7. The fastener 27 employed to implement the invention is not limited to the mechanical fastener as employed in the illustrated embodiment and it is also possible to employ the well-known fastener of adhesive type. In the case of the adhesive fastener, the loop fastener 29 will be unnecessary.

With the diaper 1 according to the invention, the pair of rear wings 13 each comprising the inner and outer layer members 21, 22 facilitate both surfaces of the wings to provide a comfortable touch. However, the wings may be formed by any one of these inner and outer layer members so far as a touch of the wings is considered to be not so important. It is also possible to implement the invention by attaching the fasteners 27 to the pair of front wings 12 rather than to the pair of rear wings 13.

Each of the wings of the inventive garment comprises the extendible sheet members and the elastically stretchable strips respectively extending along the upper and lower edges of these sheet members wherein these sheet members and strips are bonded together with no tension. Before as well as after its extension, the wing of such construction is free from the formation of gathers which would deteriorate feeling to wear the garment as has conventionally been observed in the wing of prior art.

The relatively rigid and non-stretchable fastener member is attached to the distal end of each wing so as to extend transversely of the elastically stretchable strips. The wing can be stretched uniformly over its entire width as the wing is stretched with this fastener member held by the wearer or the helper without any apprehension that the wing and the end flap of the garment connected to the wing might be curved outwardly of the wearer's body.

## Claims

1. Disposable garment (1) comprising a main body (6) having a transverse direction defining the wearer's waist regions and a longitudinal direction orthogonal to said transverse direction, said main body being assembled from a liquid-permeable topsheet (2), a liquid-impermeable backsheet (3) and a liquid-absorbent core (4) disposed between these two sheets, said main body having front and rear waist regions (7, 8) with respect to said longitudinal direction and a crotch region (9) extending between these waist regions; said main body (6) having side edges opposed to each other in the transverse direction and a pair of wings (12, 13) extending outward of said main body in said transverse direction from the side edges of the main body in said rear waist region; and fastener means (27) attached to respective distal ends (26B) of said wings (12, 13) for separably coupling said front and rear waist regions (7, 8) to each other wherein said wings respectively comprise layer members (21, 22) being extendible in said transverse direction and elastically stretchable strips (23, 24) placed along upper and lower edges (28A, 28B) of said layer members extending in said transverse direction and having a stretch stress higher than that of said layer members; and
said fastener means (27) are non-stretchable and more rigid than said wings (12, 13) wherein said fastener means are attached to said distal ends (26B) of said wings so as to extend in said longitudinal direction and to overlap said elastically stretchable strips (23, 24) on both the upper and lower edges of said layer members; **characterised in that**
said wings are formed separately from the main body, and fixed thereto by bonding means and said elastically stretchable strips (23, 24) are extending in said transversal direction without tension.

2. The garment according to Claim 1, wherein said fastener means (27) extend in said longitudinal direction across the entire widths of said wings.

3. The garment according to Claim 1 or 2, wherein said elastically stretchable strips (23, 24) comprise a hot melt adhesive agent.

## Patentansprüche

1. Wegwerf-Kleidungsstück (1) mit einem Hauptkörper (6) mit einer Querrichtung, die die Hüftbereiche des Trägers festlegt, und mit einer zur Querrichtung orthogonalen Längsrichtung, wobei der Hauptkörper aus einer flüssigkeitsdurchlässigen Oberlage (2), einer flüssigkeitsundurchlässigen Rücklage (3) und einem zwischen diesen beiden Lagen befindlichen flüssigkeitsabsorbierenden Kern (4) aufgebaut ist und einen vorderen sowie einen hinteren Hüftbereich (7, 8) bezüglich der genannten Längsrichtung und einen zwischen den Hüftbereichen verlaufenden Schrittbereich (9) aufweist, wobei der Hauptkörper (6) einander in Querrichtung gegenüberliegende Seitenkanten und ein Paar Flügel (12, 13) aufweist, die von den Seitenkanten des Hauptkörpers in dem hinteren Hüftbereich in Querrichtung nach auswärts des Hauptkörpers verlaufen, und wobei an den entsprechenden fernen Enden (26B) der Flügel (12, 13) Befestigungseinrichtungen (27) angebracht sind, um den vorderen und den hinteren Hüftbereich (7, 8) trennbar miteinander zu verbinden,
wobei die Flügel jeweils Lagenelemente (21, 22), die in der genannten Querrichtung ausziehbar sind, und elastisch dehnbare Streifen (23, 24), die entlang oberer und unterer Kanten (28A, 28B) der in Querrichtung verlaufenden Lagenelemente angeordnet sind und eine höhere Dehnspannung als die Lagenelemente aufweisen, enthalten, und
die Befestigungseinrichtungen (27) undehnbar und steifer als die Flügel (12, 13) sind und an den fernen Enden (26B) der Flügel so befestigt sind, daß sie in die genannte Längsrichtung verlaufen und sich sowohl auf der oberen als auch der unteren Kante der Lagenelemente mit den elastisch dehnbaren Streifen (23, 24) überlappen,
**dadurch gekennzeichnet, daß**
die Flügel getrennt vom Hauptkörper ausgebildet sind und an diesem mittels einer Bondeinrichtung befestigt sind und die elastisch dehnbaren Streifen (23, 24) in der genannten Querrichtung ohne Spannung verlaufen.

2. Kleidungsstück nach Anspruch 1, wobei die Befestigungseinrichtungen (27) in der genannten Längsrichtung über die gesamte Breite der Flügel verlaufen.

3. Kleidungsstück nach Anspruch 1 oder 2, wobei die elastisch dehnbaren Streifen (23, 24) ein Heißschmelz-Haftmittel enthalten.

## Revendications

1. Vêtement jetable (1) comprenant un corps principal (6) qui présente une direction transversale définissant les régions de ceinture du porteur et une direction longitudinale orthogonale à ladite direction transversale, ledit corps principal étant assemblé à partir d'une feuille supérieure (2) perméable aux liquides, d'un feuille arrière (3) imperméable aux liquides et d'un noyau (4) absorbant les liquides disposé entre ces deux feuilles, ledit corps principal comprenant en outre des régions de ceinture avant et arrière (7, 8) par référence à ladite direction longitudinale et une région d'entrejambes (9) s'étendant entre ces régions de ceinture, et ledit corps principal (6) comportant des bords latéraux opposés l'un à l'autre dans la direction transversale et une paire d'ailes (12, 13) s'étendant vers l'extérieur dudit corps principal dans ladite région de ceinture arrière; et des moyens de fixation (27) attachés à des extrémités distales respectives (26B) desdites .ailes (12, 13) pour -accoupler l'une à l'autre, de façon séparable, lesdites régions de ceinture avant et arrière (7, 8),
dans lequel lesdites ailes comprennent respectivement des éléments en couches (21, 22) qui sont extensibles dans ladite direction transversale, et des bandes étirables élastiquement (23, 24) qui sont placées le long de bords supérieurs et inférieurs (28A, 28B) desdits éléments en couches s'étendant dans la direction transversale et dont la déformation à l'étirage est plus élevée que celle desdits éléments en couches; et
lesdits moyens de fixation (27) sont non étirables et plus rigides que lesdites ailes (12, 13), lesdits moyens de fixation étant attachés auxdites extrémités distales (26B) desdites ailes de façon à s'étendre dans ladite direction longitudinale et à recouvrir lesdites bandes étirables élastiquement (23, 24) sur les bords tant supérieurs qu'inférieurs desdits éléments en couches,
**caractérisé en ce que**
lesdites ailes sont formées séparément dudit corps principal et y sont fixées par des moyens de liaison, et lesdites bandes étirables élastiquement (23, 24) s'étendent sans tension dans ladite direction transversale.

2. Vêtement selon la revendication 1, dans lequel lesdits moyens de fixation (27) s'étendent dans ladite direction longitudinale transversalement aux largeurs entières desdites ailes.

3. Vêtement selon la revendication 1 ou 2, dans lequel lesdites ailes extensibles élastiquement (23, 24) comprennent un agent adhésif à fusion à chaud.
